Europäisches Patentamt

European Patent Office

Office européen des brevets

(19)

(11) **EP 1 484 706 A2**

(12) **EUROPEAN PATENT APPLICATION**

(43) Date of publication:
08.12.2004 Bulletin 2004/50

(51) Int Cl.7: **G06F 19/00**

(21) Application number: **04013045.2**

(22) Date of filing: **02.06.2004**

| | |
|---|---|
| (84) Designated Contracting States:<br>**AT BE BG CH CY CZ DE DK EE ES FI FR GB GR**<br>**HU IE IT LI LU MC NL PL PT RO SE SI SK TR**<br>Designated Extension States:<br>**AL HR LT LV MK**<br><br>(30) Priority: **03.06.2003 JP 2003158506**<br><br>(71) Applicant: **Hitachi Ltd.**<br>**Tokyo (JP)**<br><br>(72) Inventors:<br>• **Miyamoto, Tetsuro c/o Hitachi Ltd., I.P. Group**<br>**Tokyo 100-8220 (JP)** | • **Togashi, Shigenori c/o Hitachi Ltd., Intel.Prop.Gr**<br>**Chiyoda-ku, Tokyo 100-8220 (JP)**<br>• **Miyake, Ryo c/o Hitachi Ltd., Intel.Prop. Group**<br>**Chiyoda-ku, Tokyo 100-8220 (JP)**<br>• **Sasaki, Naoya c/o Hitachi Ltd., Intel.Prop.Group**<br>**Chiyoda-ku, Tokyo 100-8220 (JP)**<br><br>(74) Representative: **Beetz & Partner Patentanwälte**<br>**Steinsdorfstrasse 10**<br>**80538 München (DE)** |

(54) **Method for estimating effect of working substance and screening method**

(57)    The object of the present invention is to estimate the effect of working substances on actual living organisms such as cells and tissues. The present invention provides a method for estimation of the effect of working substance which includes the following steps (a) and (b):

(a) a step of contacting a working substance with a model cell to measure a cell activity value of the model cell, and

(b) a step of estimating the effect of the working substance on the target living organism when the working substance is allowed to act on the target living organism, using the cell activity value measured at the step (a) as an input value.

FIG.2

EP 1 484 706 A2

**Description**

Background of the Invention

The present invention relates to a method for estimation of the effect of a working substance which can be employed, for example, for evaluating effect and toxicity of chemical substances which are candidates for drugs in research and development of new drugs and searching the chemical substances effective as drugs, and further relates to a screening method.

Recently, mechanisms of gene expression and action of proteins in the cells are being elucidated by the application of the technologies such as DNA analysis and protein analysis, and the resulting information is utilized for treatment of diseases or development of drugs. Particularly, in the development of drugs, more effective development of new drugs is expected to be performed by searching the compounds (ligands) which bind with and act on receptor proteins which participate in symptoms.

Actually, some methods have been conducted, one of which is as follows. In order to screen a compound which shows a high activity for the target receptor protein among candidate (lead) compounds for drugs, genes of the target receptor protein are introduced into an easily handleable experimental cell (for example, oocyte) to forcedly express a receptor protein, and various lead compounds are administered thereto to investigate the activity of the lead compounds.

The action of living cells and living tissues or organs depends on many proteins in addition to the target protein and on interactions such as chemical reactions. The above-mentioned screening merely evaluates binding and action of the target receptor protein and the lead compound, and cannot observe the action brought about as a result of many interactions such as inherent cell tissues. Therefore, even if a lead compound which binds with and acts on the target receptor protein is found by the above-mentioned method, the compound cannot necessarily be concluded to be effective as a drug. In many cases, a lead compound screened as a candidate is again evaluated at the stages of clinical or non-clinical tests. At the non-clinical or clinical tests, characteristics of the lead compound such as side effects or pharmacokinetics (absorption, distribution, metabolism, excretion) are evaluated.

However, actual cells are difficult to handle as compared with experimental cells. Moreover, the non-clinical and clinical tests require much const and time. Therefore, re-evaluation of the screened lead compound as a candidate is difficult, and besides re-evaluation of many lead compounds require high cost.

In view of the above problems, the present invention provides a method for estimation of effects of working substances which can estimate the effects of the working substances in real living organisms such as cells and tissues, and a method for screening the working substances, and further provides a program for estimating the effects and a system for providing a screening service.

Brief Summary of the Invention

The present invention which has attained the above object relates to a method for estimating the effect of a working substance including the following steps (a) and (b):

    (a) a step of contacting a working substance with a model cell to obtain the results of measurement of cell activity value of the model cell, and
    (b) a step of estimating the effect of the working substance on a target living organism when the working substance is allowed to act on the target living organism, using the cell activity value measured at the step (a) as an input value.

In the above step (b), a mathematical model showing the effect of the working substance on the model cell can be used. Furthermore, in the above step (b), there can be used a database which stores the cell activity value of the model cell in the case of contacting the working substance and the effect of the working substance on the target living organism in the case of contacting the working substance showing the said cell activity value with the target living organism which are related to each other.

Furthermore, the present invention provides a computer program which allows a computer to perform the method of estimating the effect of the working substance according to the present invention. The computer program of the present invention controls the operation of a computer having an operation device such as CPU and a storage device such as memory or hard disc, thereby to estimate the effect of the working substance on the target living organism.

Furthermore, the present invention can provide a system of providing a screening service for the working substances which utilizes the computer program of the present invention.

Other objects, features and advantages of the invention will become apparent from the following description of the embodiments of the invention taken in conjunction with the accompanying drawings.

Brief Description of the Several Views of the Drawings

FIG. 1 schematically shows one example of the whole construction of the screening system to which the present invention is applied.

FIG. 2 is a diagram of measurement of total cell current of Xenopus oocyte by a membrane potential clamp method.

FIG. 3 shows characteristic curves showing one example of the results of measurement of total cell current of Xenopus oocyte by membrane potential clamp method.

FIG. 4 is a schematic view of a cell having ion channels.

FIG. 5 schematically shows another embodiment of the screening system to which the present invention is applied.

FIG. 6 schematically shows another embodiment of the screening system to which the present invention is applied.

FIG. 7 schematically shows another embodiment of the screening system to which the present invention is applied.

FIG. 8 schematically shows another embodiment of the screening system to which the present invention is applied.

Detailed Description of the Invention

The present invention will be explained in detail below referring to the drawings.

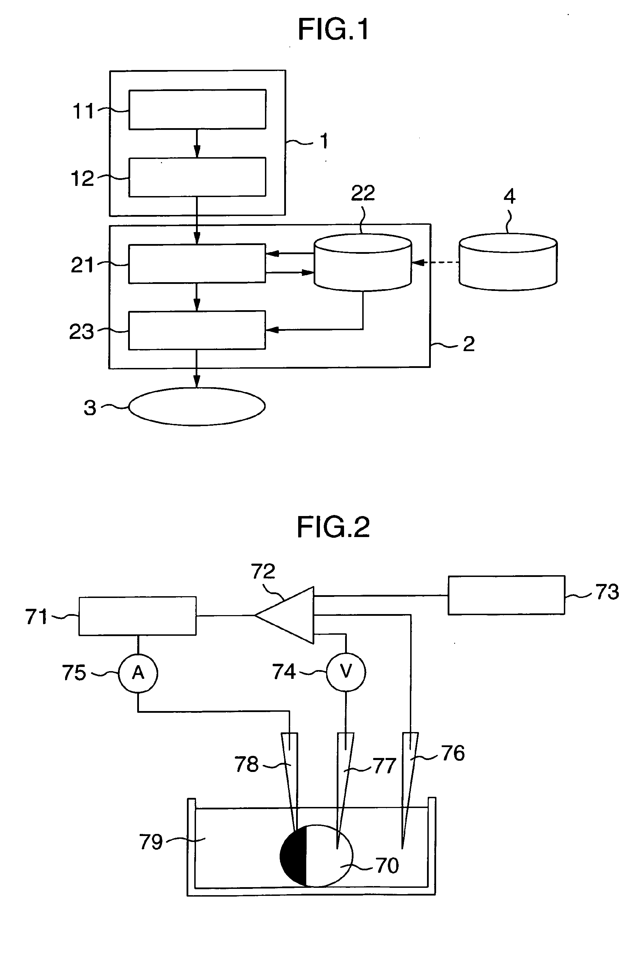

FIG. 1 schematically shows one example of the whole construction of a screening system to which the present invention is applied. As shown in FIG. 1, the screening system comprises a lead compound activity evaluating system 1 which measures the activity of a working substance (hereinafter referred to as "lead compound") to be evaluated using an experimental cell and a cell reaction estimating system 2 which estimates the reaction in a target cell to be really evaluated on the basis of the activity data obtained in the lead compound activity evaluating system 1.

[Lead compound activity evaluating system]

The lead compound activity evaluating system 1 is a system which realizes a step of measuring a cell activity value of a model cell by allowing the lead compound to contact with the model cell. The lead compound activity evaluating system 1 is provided with a computer in which the results of lead compound activity evaluation test (data showing the cell activity value) are input. Furthermore, the computer may be provided with a storage device such as a memory or a database which stores the date showing the cell activity value, a display device which displays the data, an output device which outputs the data, and a transmitting device which transmits the data to the outside.

As the "model cell" here, mention may be made of, for example, cultured animal cells such as Xenopus oocyte, HEK cells, COS cells, and CHO cells. The model cells in the lead compound activity evaluating system 1 are not limited to the above examples, and any cells can be used.

Furthermore, genes which code the target protein to be evaluated are previously introduced into the model cells. The genes may be any of genomic DNA, RNA, cDNA and cRNA which code the target protein to be evaluated. Moreover, the genes may be introduced into the model cells in such a form as containing cis-arrangements such as enhancer and promoter.

The "lead compound" is not particularly limited, and examples thereof are proteins, nucleic acids, high molecular organic compounds, low molecular organic compounds, inorganic compounds and composite compounds of these compounds.

The flow of operation in the lead compound activity evaluating system 1 will be explained. First, in a gene introduction expression step 11, for example, Xenopus oocyte is provided as a model cell, and cRNA which codes the protein to be evaluated on activity of lead compound (such as ion channel or receptor) is introduced into the above cell to express the protein to be evaluated. In case the target protein to be evaluated is expressed to some extent in normal state, the gene introduction expression step 11 may not be needed.

Next, a cell activity measuring step 12 is carried out. The cell activity measuring step 12 can perform electrophysiological analysis, metabolic physiological analysis, immunological analysis, or the like. As the electrophysiological analysis, for example, measurement of total cell current can be performed by two-electrode membrane potential clamp method as shown in FIG. 2.

As shown in FIG. 2, a potential measuring electrode 77 using a glass tube filled with a conductive solution (such as KCl solution) and a current measuring electrode 78 are thrust into a Xenopus oocyte 70 in an extracellular solution 79, and an indifferent electrode 76 is provided in the extracellular solution. A membrane potential measuring circuit 74 is connected to the potential measuring electrode 77, and the membrane potential is kept at a certain value designated with a potential generation circuit 73 due to feedback by a negative feedback circuit 72 and an amplification circuit 71. The total cell current is measured by a current measuring circuit 75 connected to the current measuring electrode 78.

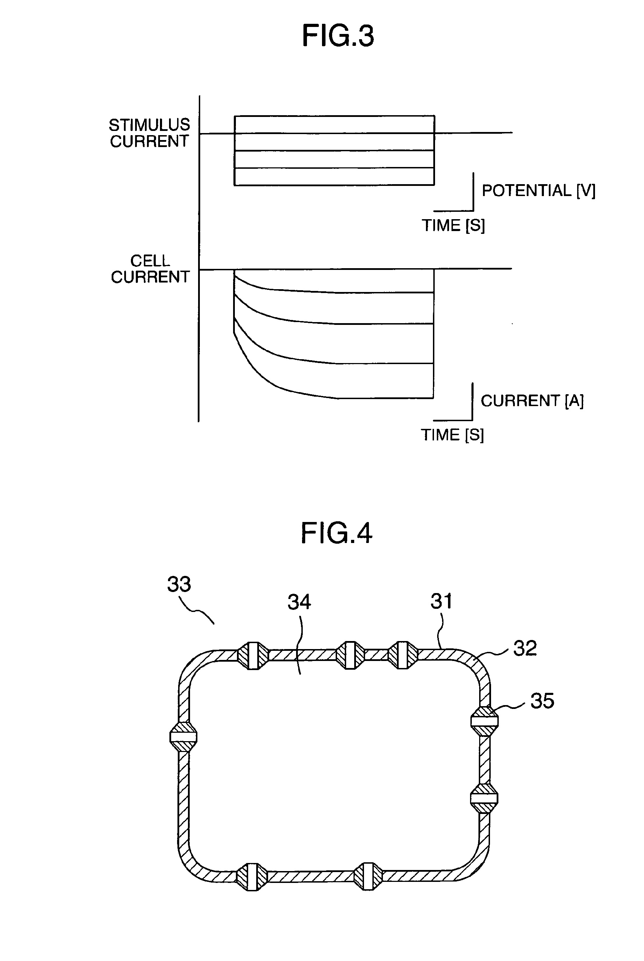

In the cell activity measuring step 12, the cell current measurement is carried out with changing the conditions such as composition of extracellular solution 79, concentration of the lead compound added to the solution and stimulus potential applied by the potential generation circuit 73, and the relationship of membrane potential - concentration of lead compound - total cell current. One example of the results of measurement of cell current is shown in FIG. 3.

In the lead compound activity evaluating system 1, data showing the cell activity value can be obtained as the results of the lead compound activity evaluation test measured as mentioned above. The resulting data are stored, for example, in the storage device of the computer, and output to the cell reaction system 2 mentioned hereinafter. Further, the resulting data may be converted to an input format by a program of converting to the input format in accordance with the cell reaction system 2 mentioned hereinafter, and then the data are stored.

[Cell reaction estimating system]

The cell reaction estimating system 2 estimates the reaction in a living organism using the cell activity value measured in the lead compound activity evaluating system 1. The cell reaction estimating system 2 is provided with a computer in which is installed a program carrying out the estimation of reaction in the target living organism using the data obtained in the lead compound activity evaluating system 1 as an input value. The computer in the cell reaction estimating system 2 may be the same as or different from the computer in the lead compound activity evaluating system 1.

Here, "target living organisms" include all living organisms, for example, tissues and organs such as cells, skins, vessels and muscles, and combinations thereof.

In the cell reaction estimating system 2, a mathematical model is prepared for in a mathematical model estimating step 21 before carrying out the estimation of the cell reaction. For example, in the case of carrying out the electrophysiological analysis in the lead compound activity measuring system, the following mathematical model is prepared.

[Preparation of mathematical model]

In carrying out the estimation operation, a mathematical model of a cell to be estimated is prepared. For example, a cell 31 as schematically shown in FIG. 4 is exemplified. The cell 31 is parted into interior and exterior with a cell membrane 32 comprising a lipid bilayer. The exterior of the cell membrane 32 is filled with an extracellular solution 33 and the interior is filled with a cytoplasm 34. Several ion channels 35 which are membrane piercing proteins and selectively pass ions are expressed on the cell membrane, and ions contained in the extracellular solution 33 and the cytoplasm 34 pass through the ion channels 35 and go in and out the cell. Furthermore, when there is a difference in concentration of ions in interior and exterior of the cell 31, a potential difference (membrane potential) is generated.

Assuming that n kinds of ion channels and m kinds of ions are present in the cell 31 and when the internal and external concentrations of ion j are shown by $[C_j]_{in}$ and $[C_j]_{ex}$, the rate of change $[d[C_j]_{in}/dt]_i$ of concentration of ion j per unit time due to passing through the ion channels i can be expressed, for example, as follows.

$$\left[ \frac{d[C_j]_{in}}{dt} \right]_i = f_{ij}([C_j]_{in}, [C_j]_{ex}, E, T) \cdot p_{ij} \quad \cdots \cdots \quad (1)$$

The above formula (1) is a function which shows the flow rate of ion j when the ion channels i are opened and depends on the ion concentrations $[C_j]_{in}$ and $[C_j]_{ex}$ in interior and exterior of the cell, the membrane potential E and the temperature T. The symbol $P_{ij}$ is a function showing a probability of opening of the gates of ion channels i, and, for example, when the ion channels have a gate mechanism of membrane potential dependence type, it is shown by a differential equation as shown below.

$$\frac{dp_{ij}}{dt} = g_{ij}(p_{ij}, E, T) \tag{2}$$

The change of concentration of ion j in the total cells, namely, $[d[C_j]_{in}/dt]_{total}$, can be shown by the following formula (3).

$$\left[\frac{d[C_j]_{in}}{dt}\right]_{total} = f_{1j}p_{1j} + f_{2j}p_{2j} + \cdots + f_{nj}p_{nj} \quad \cdots \cdots (3)$$

Furthermore, when cell volume is indicated by V and valence of the ion j is indicated by $z_j$, the change of potential due to movement of ion j, namely, $dE_j/dt$, can be shown by the following formula (4).

$$dE_j/dt = E_j(d[C_j]_{in}/dt, Z_j, V) \tag{4}$$

The membrane potential E in the total cells can be shown by the following formula (5).

$$dE/dt = E_1 + E_2 + \cdots + E_m \tag{5}$$

As mentioned above, when simultaneous differential equations are prepared on all of the ion concentration, ion channel and membrane potential, behavior of cells under some conditions can be simulated by carrying out the numerical integration.

These expression models of differential equations on each of ions and ion channels or information on the ions and ion channels which constitute the cells are previously registered in the mathematical model database 22 in the cell reaction estimating system 2 of FIG. 1. It is desired that not only the information is prepared by users, but also many mathematical models of living organisms are utilized by linking through network the information with, for example, living organism physiological model database 4 published on internet.

The mathematical model when electrophysiological analysis is carried out in the lead compound activity measuring system 1 is explained above. When a metabolic physiological analysis is carried out in the lead compound activity measuring system 1, there can be prepared a mathematical model disclosed, for example, in "Glycolysis in Bloodstream Form Trypanosoma brucei Can Be Understood in Terms of the Kinetics of the Glycolytic Enzymes" (Barbara M Bakker, et al, The Journal of Biological Chemistry, 272, 3207-3215, 1997). This mathematical model concerns with glycolytic pathway of blood pathogenic bacterium called Trypanosoma brucei.

Furthermore, mathematical models of metabolism signal transfer are disclosed in http://www.cellml.org/examples/repository/ and these mathematical models can be prepared. Although parameters are not included, outline of metabolic model is disclosed in http://www.genome.ad./kegg/kegg2.html.

[Simulation]

Successively, effect of the lead compound on the target living organism is estimated in the cell reaction estimating system 2 using the cell activity value measured in the lead compound activity evaluating system 1 and the mathematical model prepared as mentioned above. Specifically, as explained below, a software in which the above mentioned mathematical model is mounted is constructed, and the effect of the lead compound on the target living organism is estimated by a computer in which this software (hereinafter sometimes referred to as "simulation program") is installed, using the cell activity value as an input value.

Specifically, in the cell reaction estimating system, when the objective protein is ion channel, for example, mathematical model formulas of ion channels represented in the form of the above formulas (1) and (2) on the objective ion channels are identified in comparison with control test on oocyte in which cRNA is not introduced.

In the case of the lead compound being a ligand acting on the gate mechanism, it can be expressed in the form of the formula (2) depending on the ligand concentration [L], for example, as shown below.

$$\frac{dp_{ij}}{dt} = g_{ij}(p_{ij}, E, T, [L]) \tag{2'}$$

The formula (2') contains unknown parameter regarding the relation with the ligand concentration [L].

Therefore, the corresponding formulas (1) and (2') are selected from the mathematical model database 22, and test conditions and results are input therein and the parameters of (2') are optimized so as to conform to the experimental results. Furthermore, for example, in case the action mechanism is unclear and the forms of functions of (1) and (2') are unknown, some candidate formulas are listed up from the mathematical model database 22, and a formula optimum to the test results may be selected.

When there is no corresponding formula and the mathematical model cannot be identified from the results of measurement in the cell activity measuring step 12, it is preferred to carry out a detailed analysis of simple ion channel by patch-clamp method or the like, and directly identify the formula.

Furthermore, new formulas and parameters obtained from these results are newly registered in the mathematical model database 22, and are utilized next time when investigation of this lead compound becomes necessary.

Next, in the simulation step 23 of FIG. 1, a simulation program of a cell which is to be really evaluated is constructed by the formulas (1)-(5), etc. which constitute the cell model registered in the mathematical model database 22.

The constructed cell simulation program is registered in the mathematical model database 22, and when investigation is carried out on this cell next time, the program can only be called out without carrying out reconstruction.

Next, the formula representing action model of the corresponding ion channel is replaced with the mathematical model formula (2') which represents the action of the ion channel-lead compound and is obtained in the mathematical model estimating step 21.

Furthermore, as the simulation conditions, there are input the initial conditions of various ion concentrations in and out of the cell in the environment of the target cell to which the lead compound is to be really administered, the temperature, the administration concentration of the lead compound, the simulation time, etc.

Finally, when the simulation program is practiced, the program carries out numerical integration for the predetermined simulation time on the simultaneous differential equations which show behaviors of various ion channels incorporated.

As a result, changes with time of the constitutive elements of the cell defined in the simultaneous differential equations (such as various ion concentration in and out of the cell, membrane potential and opening rate of channels) are calculated and these values are output as estimated reaction results 3 of the target cell.

As a result, the action processes of the lead compound are displayed as the changes with time of each of the constitutive elements of the cell, and hence there is a merit that the guideline for examination of effectiveness can be easily obtained.

[Estimation on interaction of a plurality of lead compounds]

In the above example, the effect of single lead compound on the target living organism is estimated, but various synergistic effects may be brought about by the interaction of a plurality of lead compounds. Therefore, in this system, for example, lead compound A, lead compound B and lead compound C are individually tested particularly in the lead compound activity evaluating system 1, and the results of model estimation are registered in the mathematical model database 22. As a result, by incorporating the numerical formulas of the above three action models into the simulation program in the simulation step 23, the results of interaction of a plurality of the cases of combination such as AB, BC, AC, and ABC can be examined in addition to the results of individual administration of the lead compounds A, B and C.

In this case, there is a merit that the experiments of simultaneous administration of a plurality of the lead compounds can be sharply diminished. Moreover, since the action process is clarified from the results of simulation, the effectiveness of the interaction can also be examined qualitatively.

In this example, only the ion channels are handled, but in a cell there occur various actions such as metabolisms of transporters, other receptor proteins and enzyme reaction, and migration of substances, and by adding them to the simulation program as mathematical expressions such as differential equation in the same manner as above, the results of behavior of cells including these functions can be forecasted.

[Other embodiments in this screening system]

In the screening system to which the present invention is applied, further detailed estimation operation can be performed considering the function of other proteins which are inherently expressed in a model cell as explained below.

That is, in this case, from the results of control experiments using a cell into which cRNA is not introduced in the cell activity measuring system 12, it is considered that not only the protein to be evaluated, but also other proteins which are usually contained in the cell act with the lead compound to affect the results of measurement. In this case, if it is considered that in this experimental cell, only the formulas of one kind of ion channel which can be represented in the form of the above-mentioned formulas (1) and (2) are set up, there is the possibility that exact estimation operation cannot be performed.

Therefore, in this screening system, first, in the mathematical model estimating step 21, the mathematical models equivalent to (1)-(5) are called out from the mathematical model database 22 as to the other proteins than those to be evaluated which are expressed in model cell (a known ion channel group), and using them a simulation model is constructed. Furthermore, formulas equivalent to the mathematical models (1) and (2') on the relationship between the protein to be evaluated (objective ion channels) and the concentration of lead compound are also called out from the mathematical model database 22 on the proteins other than those to be evaluated, and the formulas are added to the mathematical model.

Then, simulation is conducted using the test conditions in the cell activity measuring step 12 as initial conditions, and the parameters in the formula (2') are adjusted so as to give a minimum error between the results of the simulation and the test results in the cell activity measuring step 12. For the adjustment of parameters, there can be employed a mathematical convergence method such as Powell method or a trial and error method such as genetic algorithm.

After the mathematical model showing the relationship between the proteins other than those to be evaluated and the concentration of the lead compound is obtained in this way, the mathematical model showing the relationship between the proteins other than those to be evaluated and the concentration of the lead compound is added to the simulation program of the target living organism in the simulation step 23, and simulation is conducted.

In this case, even if the expression amount of the target protein to be evaluated is very small in the model cell, the effect of interaction between the proteins other than the target protein and the lead compound can be excluded. Therefore, the interaction between the target protein to be evaluated and the lead compound can be more accurately estimated.

[Other embodiment in this screening system]

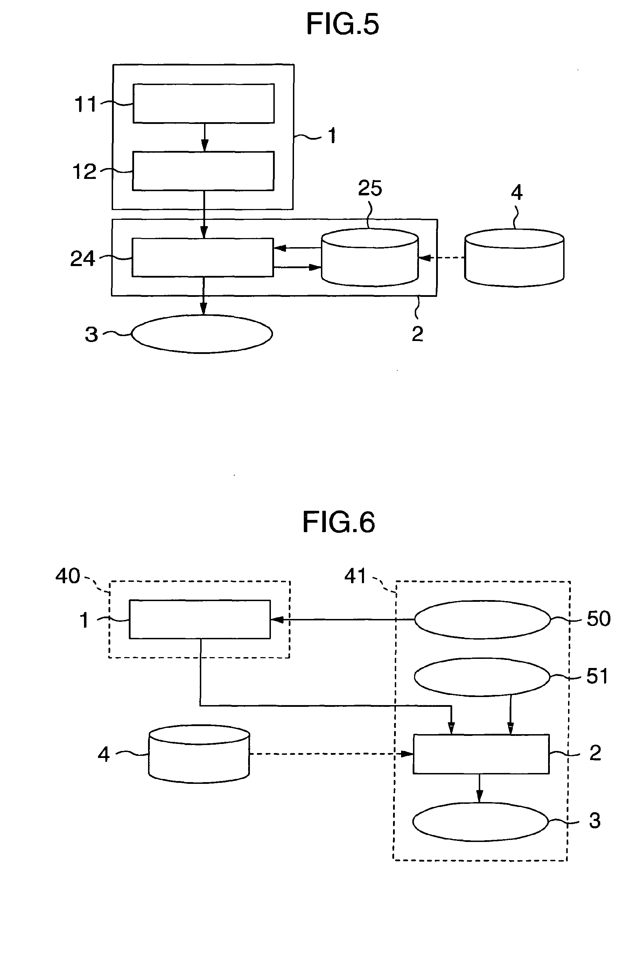

Moreover, this screening system can estimate the reaction in the target living organism by the cell reaction estimating system 2 having a cell reaction judging step 24 and the cell activity-reaction database 25 as shown in FIG. 5.

In this example, the results of the cell activity measuring step 12 carried out with a model cell in the past and the results of the lead compound administration test are correlated to each other on every test condition, and stored in the cell activity-reaction database 25. Here, the results of the cell activity measuring step 12 carried out with a model cell in the past are stored by relating with evaluation indexes calculated based on the evaluation functions using as parameters the items such as concentration of lead compound, changing rate of cell current and changing time of cell current.

In this screening system, the cell activity measuring step 12 is carried out in the same manner as in the above example, and the cell reaction judging step 24 is carried out using the resulting cell activity value. In the cell reaction judging step 24, evaluation indexes are calculated on the basis of the evaluation functions using as parameters the items of the cell activity measuring step 12 (concentration of lead compound, changing rate of cell current and changing time of cell current).

Next, in the cell reaction judging step 24, evaluation indexes closest to the evaluation indexes calculated from the results of the cell activity measuring step 12 are retrieved from the cell activity-reaction database 25. The results of the cell activity measuring step 12 carried out with a model cell in the past which are correlated to the retrieved evaluation indexes and the results of the lead compound administration test in the target living organism corresponding to the results of the cell activity measuring step 12 are extracted as the results of estimation of reaction.

As examples of evaluation function index, there is a simple method of subjecting to primary approximation the relation between the concentration of lead compound and the maximum value of the change of cell current and comparing the slope thereof as the evaluation index with the evaluation index of the cell activity-reaction database 25.

As another example of the evaluation function index, there is the following method: the slope of the result of the primary approximation is referred to as evaluation index A and an average time for the rate of change in cell current decreasing from the maximum value to a predetermined value is referred to as evaluation index B, then, $(A-A')^2 + w(B-B')^2$ is calculated using evaluation indexes A' and B' and weighted constant w in the cell activity - reaction database 25, and an evaluation function index when the calculated value becomes minimum is retrieved.

Alternatively, coincidence degree of the evaluation index calculated from the results of the cell activity measuring step 12 and the evaluation index stored in the cell activity-reaction database 25 may be retrieved in the cell reaction judging step 24. In this case, for example, the evaluation indexes showing the coincidence degree exceeding a predetermined standard can be synoptically displayed as the results of estimation of reaction in order of the height of the coincidence degree. That is, the results of the cell activity measuring step 12 carried out with a model cell in the past which are correlated with the evaluation indexes of high coincidence degree and the results of the lead compound administration test in the target living organism corresponding thereto can be displayed in order of the height of coincidence degree.

In this case, distribution of the evaluation indexes of the results of the lead compound administration test in the target living organism can be displayed. Thus, propriety of the estimation results can be evaluated according to the scattering degree of the distribution of the estimation results.

Particularly, in this screening system, there is no need to prepare the mathematical model mentioned above and besides there is no need to carry out simulation from the results of the cell activity measuring step 12. Therefore, the effect of the lead compound in the target living organism can be more simply estimated.

[Business model 1 using this screening system]

A method of providing a screening service of lead compounds in development of drugs, etc. using the above-mentioned screening system will be explained referring to FIG. 6.

FIG. 6 shows a diagram of this screening service. This service is composed of a service provider 40 and a service user 41. The service provider 40 has the above lead compound activity evaluating system 1. A computer and a storage device, and the cell reaction estimating system 2 consisting of a program which estimates various reactions in a cell and a database are provided for or lent to the service user 41 by the service provider 40.

According to this service, first the service user 41 shows experimental cell testing conditions 50 to the service provider 40 and requests the screening service.

The experimental cell test conditions 50 include information on the lead compound to be screened (chemical formula, etc.), and information on the screening conditions (administration concentration of the lead compound, etc.) and on the protein to be evaluated. Particularly, the experimental cell test conditions 50 are preferably information excluding the information on the lead compound to be screened. When the information on the lead compound to be screened is not disclosed as the experimental cell test conditions 50, for example, the service user 41 separately provides for the service provider 40 the lead compound having a control number. Thus, in this service, the information on the lead compound can be concealed from the others including the service provider 40.

Next, the service provider 40 who is requested to carry out the screening conducts an activity evaluation test of the lead compound in a model cell by the above-mentioned lead compound activity evaluating system 1 on the basis of the experimental cell test conditions 50. The service provider 40 converts the obtained test results to an input format for the cell reaction estimating system 2 possessed by the service user 41 and transmits it to the service user 41.

Next, the service user 41 inputs the transmitted data and target cell estimating conditions 51 in the cell reaction estimating system 2.

The target cell estimating conditions 51 are information on the target living organism such as cell to be examined by the service user 41. Examples of the information are kind of the target living organism, initial conditions and information as to other lead compounds different from the lead compound requested to be screened.

The service user 41 estimates the results of administration of the lead compound in the target living organism by the cell reaction estimating system 2 as mentioned above to obtain reaction estimation result 3 of the target living organism. In the method of providing the screening service explained in this example, the service provider 40 provides or lends the cell reaction estimating system 2 for or to the service user 41. Thus, the service provider 40 can only carry out the activity evaluation test on the lead compound using a model cell and provide the results for the service user 41 in a predetermined format, and does not need to estimate the results of administration of the lead compound in the target living organism.

On the other hand, the service user 41 can outsource the activity evaluating test on the lead compound using a model cell, and can perform the screening of drugs and the like at low cost. Especially, the service user 41 can utilize this service even if the information on the lead compound is concealed, and can inhibit leakage of the information on the lead compound and can utilize this system without anxiety.

[Business model 2 using this screening system]

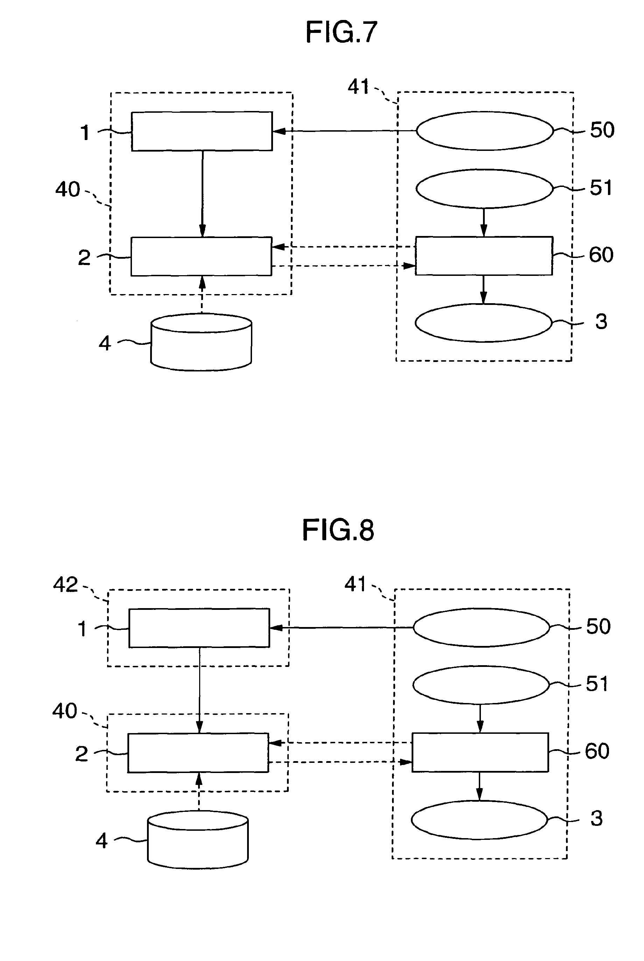

Another method of providing a screening service of lead compounds in the development of drugs, etc. using the above-mentioned screening system will be explained referring to FIG. 7.

FIG. 7 shows a diagram of this screening service. This service is composed of a service provider 40 and a service user 41. The service provider 40 has the lead compound activity evaluating system 1 and the cell reaction estimating system 2 consisting of a computer and a storage device, a program which estimates various reactions in a cell, and a database. Furthermore, the service user 41 has a communication terminal 60 by which the service user 41 can access the cell reaction estimating system 2 controlled by the service provider 40.

According to this service, first the service user 41 shows experimental cell test conditions 50 to the service provider 40 and requests to carry out the screening service in the same manner as in screening service shown in FIG. 6.

Next, the service provider 40 who is requested to carry out the screening conducts an activity evaluation test of the lead compound in a model cell by the above-mentioned lead compound activity evaluating system 1 on the basis of the experimental cell test conditions 50 in the same manner as in the screening service shown in FIG. 6. The service provider 40 converts the obtained test results to an input format for the cell reaction estimating system 2 and inputs it in the cell reaction estimating system 2. Furthermore, the service provider 40 gives the service user 41 a certain authority to access the cell reaction estimating system 2. When the authority to access is given by the service provider 40, the service user 41 can operate the cell reaction estimating system 2 through network.

Next, the service user 41 inputs the target cell estimating conditions 51 in the cell reaction estimating system 2 through network using the communication terminal 60. The service user 41 can operate the cell reaction estimating system 2 through network, and can estimate the results of administration of the lead compound in the target living organism. The results of the estimation are sent to the communication terminal 60 through network as the reaction estimation results 3 of the target living organism, and the service user 41 can utilize them. A series of these communication data through network are desirably concealed from those other than the service user 41 by encoding.

In this service, the service provider 40 has the cell reaction estimating system 2 and allows the service user 41 to utilize the cell reaction estimating system 2 through network. By this construction, the service provider 40 can more easily perform maintenance and control of the computer and the storage device constituting the cell reaction estimating system 2 and the database as compared with the case where the cell reaction estimating system is provided for or lent to many service users 41. In other words, in this service, since the service user 41 utilizes the cell reaction estimating system possessed by the service provider 40, there is no need to perform maintenance and control of the computer, the storage device, the program and the database which constitute the cell reaction estimating system 2. Therefore, this service is very easy to utilize for the service user 41.

Furthermore, the service provider 40 can make contracts in various forms with the service user 41. That is, in this service, the service provider 40 can minutely set the scope of the right to access the cell reaction estimating system 2, the accessing period, the scope of utilizable database, etc. depending on the form of contract, and can provide diverse services. In other words, the service user 41 can make contracts with the service provider 40 depending on the object for utilization of this service and can effectively utilize this service.

[Business model 3 using this screening system]

Another method of providing a screening service of lead compounds in the development of drugs, etc. using the above-mentioned screening system will be explained referring to FIG. 8.

FIG. 8 shows a diagram of this screening service. This service is composed of a service provider 40, a service user 41 and a lead compound administration tester 42. The service provider 40 has a cell reaction estimating system 2 comprising a computer, a storage device, a program which estimates various reactions in a cell and a database. Furthermore, the service user 41 has a communication terminal 60 by which the service user 41 can access the cell reaction estimating system 2 controlled by the service provider 40. The lead compound administration tester 42 has a lead compound activity evaluating system 1.

According to this service, first the service user 41 shows to the lead compound administration tester 42 the same experimental cell test conditions 50 as in the screening service shown in FIG. 4, and requests a lead compound administration test.

Next, the lead compound administration tester 42 carries out an activity evaluation test of the lead compound in a model cell based on the request by the service user 41. The lead compound administration tester 42 converts the results of the lead compound administration test to an input format for the cell reaction estimating system 2 and transmits it to the service provider 40. The results of the lead compound administration test may be transmitted as they are to the service provider 40 without carrying out the conversion to the input format for the cell reaction estimating system 2. In this case, the results of the lead compound administration test may be converted to the input format on the side of the service provider 40.

Next, the service provider 40 estimates the results of lead compound administration in the target living organism by the cell reaction estimating system 2 as mentioned above using the results obtained from the lead compound administration tester 42, thereby obtaining the reaction estimation results 3 of the target living organism. In this case, the service provider 40 gives the service user 41 a certain authority to access the cell reaction estimating system 2. When the authority for accessing is given by the service provider 40, the service user 41 can operate the cell reaction estimating system 2 through network.

Next, the service user 41 inputs the target cell estimating conditions 51 in the cell reaction estimating system 2 through network using the communication terminal 60. The service user 41 can operate the cell reaction estimating system 2 through network, and can estimate the results of administration of the lead compound in the target living organism. The results of the estimation are sent to the communication terminal 60 through network as the reaction estimation results 3 of the target living organism, and the service user 41 can utilize them. A series of these communication data through network are desirably concealed from those other than the service user 41 by encoding or the like.

In this service, the service provider 40 does not have the lead compound activity evaluating system 1, but has the cell reaction estimating system 2, and allows the service user 41 to utilize the cell reaction estimating system 2 through network. By this construction, the service provider 40 has no need to carry out the lead compound activity evaluation test and can effectively analyze the results of test conducted by many lead compound activity evaluation testers 42. Moreover, the service provider 40 can more easily perform maintenance and control of the computer and the storage device constituting the cell reaction estimating system 2 and the program and database as compared with the case where the cell reaction estimating system 2 is provided for or lent to many service users 41. In other words, in this service, since the service user 41 utilizes the cell reaction estimating system possessed by the service provider 40, there is no need to perform maintenance and control of the computer and storage device constituting the cell reaction estimating system 2 and the program and database. Therefore, this service is very easy to utilize for the service user 41.

Furthermore, the service provider 40 can make contract in various forms with the service user 41. That is, in this service, the service provider 40 can minutely set the scope of accessing right to the cell reaction estimating system 2, the accessing period, the scope of utilizable database, etc. depending on the form of contract, and can provide diverse services. In other words, the service user 41 can make contracts with the service provider 40 depending on the object for utilization of this service and can effectively utilize this service.

As explained in detail hereinabove, according to the present invention, there can be provided a method for estimating the effect of a working substance, a method for screening a working substance, an effect estimating program, and a screening service providing system by which the effect of working substances on actual living organisms such as cells and tissues can be estimated.

It should be further understood by those skilled in the art that although the foregoing description has been made on embodiments of the invention, the invention is not limited thereto and various changes and modifications may be made without departing from the spirit of the invention and the scope of the appended claims.

**Claims**

**1.** A method for estimation of an effect of a working substance on a target living organism which includes the following steps (a) and (b):

(a) a step of contacting a working substance with a model cell to obtain the results of measuring a cell activity value of the model cell, and
(b) a step of estimating the effect of the working substance on the target living organism in the case of allowing the working substance to act on the target living organism, using the cell activity value measured at the step (a) as an input value.

**2.** A method for estimation according to claim 1, wherein in the step (b) the effect on the target living organism is estimated on the basis of a mathematical model showing the effect of the working substance on the model cell.

**3.** A method for estimation according to claim 2, wherein the mathematical model is an electrophysiological model of the model cell.

**4.** A method for estimation according to claim 1, wherein in the step (b) the effect on the target living organism is estimated using a database in which the cell activity value of the model cell upon contacting with the working substance and the effect on the living organism when the working substance showing said cell activity value is contacted with the living organism are stored with being related to each other.

**5.** A method for estimation according to claim 1, which additionally includes, before the step (a), a step of contacting the working substance with the model cell to measure the cell activity value of the model cell.

**6.** A method for estimation according to claim 1, which additionally includes a step of outputting the results of the measurement after the step (b).

**7.** A computer program which includes the following steps and estimates an effect of a working substance on a target living organism:

(a) a step of contacting a working substance with a model cell to obtain the results of measuring a cell activity value of the model cell, and

(b) a step of estimating the effect of the working substance on the target living organism in the case of allowing the working substance to act on the target living organism, using the cell activity value measured at the step (a) as an input value.

8. A computer program according to claim 7, wherein in the step (b) the effect on the target living organism is estimated on the basis of a mathematical model showing the effect of the working substance on the model cell.

9. A computer program according to claim 8, wherein the mathematical model is an electrophysiological model of the model cell.

10. A computer program according to claim 7, wherein in the step (b) the effect on the target living organism is estimated using a database in which the cell activity value of the model cell upon contacting with the working substance and the effect on the living organism when the working substance showing said cell activity value is contacted with the living organism are stored with being related to each other.

11. A computer program according to claim 7, which additionally includes a step of outputting the results of the measurement after the step (b).

12. A screening service providing system for a working substance which includes the following steps:

(a) a step of contacting a working substance with a model cell to obtain the results of measuring a cell activity value of the model cell, and

(b) a step of estimating the effect of the working substance on the target living organism in the case of allowing the working substance to act on the target living organism, using the cell activity value measured at the step (a) as an input value.

13. A screening service providing system according to claim 12, wherein the results obtained in the step (a) are externally obtained.

14. A screening service providing system according to claim 12, wherein in the step (b) the effect on the target living organism is estimated using a database in which the cell activity value of the model cell upon contacting with the working substance and the effect on the living organism when the working substance showing said cell activity value is contacted with the living organism are stored with being related to each other.

# FIG.1

# FIG.2

# FIG.3

STIMULUS
CURRENT

POTENTIAL [V]

TIME [S]

CELL
CURRENT

CURRENT [A]

TIME [S]

# FIG.4

33

34

31

32

35

# FIG.5

# FIG.6

# FIG.7

# FIG.8